# EUROPEAN PATENT SPECIFICATION

(11) **EP 2 305 139 B1**
(45) Date of publication and mention of the grant of the patent: **10.05.2017**
(21) Application number: 10010027.0
(22) Date of filing: 21.02.2001
(51) Int. Cl.: A61B 17/115, A61B 17/072, A61B 17/11

(54) **AN ELECTROMECHANICAL DRIVER AND REMOTE SURGICAL INSTRUMENT ATTACHMENT HAVING COMPUTER ASSISTED CONTROL CAPABILITIES**
ELEKTROMECHANISCHE ANTRIEBSVORRICHTUNG UND FERNGESTEUERTER CHIRURGISCHER GERÄTEVORSATZ MIT COMPUTERGESTÜTZTEN STEUERUNGMÖGLICHKEITEN
MOTEUR ELECTROMECANIQUE ET FIXATION D'INSTRUMENT CHIRURGICAL DOTE DE FONCTIONS DE COMMANDES ASSISTEES PAR ORDINATEUR

(30) Priority: 22.02.2000 US 510927
(43) Date of publication of application: 06.04.2011
(62) Divisional of application: 01911011.3
(73) Proprietor: Covidien LP, Mansfield, MA 02048 (US)
(72) Inventor: Whitman, Michael, P., New Hope PA 18938 (US)
(74) Representative: Maschio, Antonio

(56) References cited:
- US-A- 5 460 182
- US-A- 5 667 517

## Description

The present invention relates generally to a medical tool comprising an electromechanical driver which transmits information to and receives information from, and thereby controls, a surgical instrument attachment, and more specifically to the mechanisms employed to provide such remote direction and information relaying between said electromechanical driver and said surgical instrument attachment.

It shall be understood at the outset, that the present invention has many applications within the field of surgery. This disclosure shall not, therefore, be read as limiting of the scope of the invention by the specific medical or surgical applications which may be described herein, as they are only used as elucidating examples of such applications in which the present invention may be employed to enhance the outcomes and/or surgical efficiency. In particular, the present disclosure is directed to embodiments used in colon surgery, and specifically to anastomosing, resecting, and stapling instruments, however, the same invention may be applied to other surgical applications in the fields of gynecological surgery, cardiovascular surgery, and general surgery.

Upon identification of cancerous or other anomalous tissue in the gastrointestinal tract, surgical intervention is often prescribed. The field of cancer surgery, and more specifically, the surgical procedure by which a section of the gastrointestinal tract which includes cancerous or anomalous tissue is resected, includes a number of uniquely designed instruments. In combination with a description of the present instrumentation and their functions, a description of the state of the art in this surgical procedure shall also be provided.

The first question which must be answered when determining how to treat gastrointestinal cancer relates to the specific location of the cancerous tissue. This is very important insofar as the instruments which are provided in the present art have limitations relating to how far they may be inserted into the gastrointestinal tract. If the cancerous tissue is too far up the colon, for example, then the standard instrumentation provided is unusable, thus requiring special accommodations. These accommodations generally increase the risk of contamination of the surrounding tissues with bowel contents, increase the length of the surgery and the corresponding need for anesthesia, and eliminate the benefits of precise anastomosing and stapling which comes from utilizing a mechanized device.

More specifically, in the event that the cancerous tissue is located at a position in the colon which is accessible by the present instrumentation, the patient's abdomen is initially opened to expose the bowel. The surgeon then utilizes a linear cutter and stapling device which cuts the tube of the colon on either side of the cancerous tissue, thereby creating two stapled ends of the bowel (a distal end which is directed toward the anus, and the proximal end which is closest to the small intestine). This is done in order to temporarily minimize contamination.

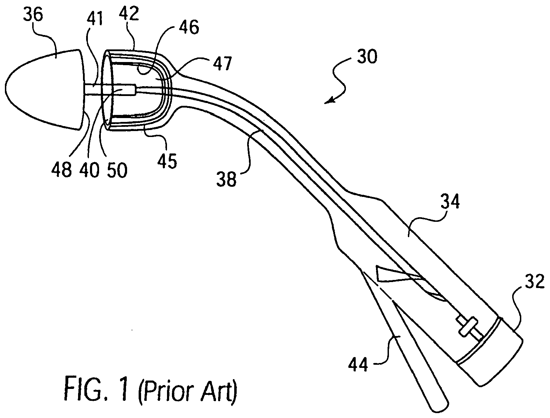

The surgeon then partially opens the proximal end and inserts the removable anvil portion of an anastomosing and stapling instrument into the exposed proximal end. This step, as well as those of the remainder of the surgical procedure, are related to the functioning of this surgical instrument. More particularly, and with respect to Figure 1, the surgeon begins by taking the instrument 30 and manually turning the dial 32 at the base of the handle 34 which causes the anvil head 36 at the opposite end to advance forward. The surgeon continues to turn the dial 32 until the anvil head 36 advances to its most extreme extended position. This manual turning requires nearly thirty full rotations. Once fully extended, the anvil head of the instrument is decoupled therefrom and is inserted into the partial opening of the proximal end such that the coupling post extends outwardly therethrough. This partial opening of the proximal end is then sutured closed. The extending shaft 38 of the anastomosing and stapling instrument 30 is then inserted and advanced into the lower colon, transanally, until the coupling stem 40 thereof extends through the stapled distal end. The surgeon then joins the coupling ends of the anvil and shaft together and begins to manually rotate the dial in the handle again, this time bringing the anvil head closer to the end 42 of the shaft.

Once the anvil head and shaft are brought close together, after the surgeon has manually rotated the dial another thirty times, a grip-style trigger 44 in the handle is manually actuated. This actuation causes a circular blade 46 to advance axially out from the tip of the shaft, and into contact with the opposing face 48 of the anvil 36. The blade cuts through the stapled-closed ends of the proximal and distal ends of the colon, thereby also cutting a new pair of ends of the proximal and distal portions of the colon. The tissue which has been severed is held in an interior volume at the end of the shaft.

In lock step with the cutting, the freshly opened ends are joined together by a series of staples 50 which are advanced through holes in the perimeter of the tip of the shaft (being pressed against and closed by the opposing face of the anvil). The coupled shaft and anvil are then withdrawn from the patient.

As with many such devices of the prior art, all of these devices are considered fully disposable, and are, in fact, thrown away after a single use. They are complicated devices, having multiple moving parts, requiring substantial structural integrity and, therefore, expense in manufacturing. The fact that they are used only once, and that no part can be used again, render the use of such devices expensive and wasteful of resources.

In addition to this failure, as can be readily observed from the preceding descriptions, the prior art devices suffer from numerous other limitations which would be desirable to overcome. These include the rigid and limited length shaft of the anastomosing and stapling instrument (which limits the portion of the gastrointestinal tract which may be treated by such a device), as well as the requirement that the surgeon manually actuate a number of different functions (including those associated with the dial and trigger of the anastomosing and stapling instrument and the multiple triggers of the cutting and stapling instrument).

Therefore, it has been a principal object of recent inventions to provide an instrument for cutting, anastomosing, and stapling, for use in gastrointestinal surgery, which reduces the waste of resources by permitting the reuse of portions thereof, can extend farther into the colon, and which are more simple to manipulate.

In particular, this prior invention, made by the present inventor comprises a surgical system as recited in claim 1 with preferred features set forth in the dependent claims. An electromechanical driver assembly is described, mounted in a handle-shaped base unit, which couples to and motivates remote surgical attachments through a flexible shaft which may also be remotely manipulated by means of a series of steering wires which are controlled within the handle as well.

First, with respect to the handle component and the flexible shaft. The handle has a pistol grip-styled design, having one or more, and preferably two, finger triggers which are independently coupled to at least one, and preferably two separate motors which each turn separate flexible drive shafts (described more fully, hereinbelow). The motors are each dual direction motors, and are coupled to a manual drive switch mounted to the top of the handle, by which the user can selectively alter the turning direction of each motor. In addition to the motor components, the handle further includes several other features, including: (1) an remote status indicator; (2) a shaft steering means; and (3) at least one additional electrical supply.

The flexible shaft comprises a tubular sheath, preferably formed of a simple elastomeric material which is tissue compatible and which is sterilizable (i.e., is sufficiently rugged to withstand an autoclave). Within the elastomeric sheath are a pair of smaller fixed tubes which each contain a flexible drive shaft which is capable of rotating within the tube. The flexible drive shaft, itself, simply must be capable of translating a torque from the motor in the handle to the distal end of the shaft, while still being flexible enough to be bent, angled, curved, etc. as the surgeon deems necessary to "snake" through the colon of the patient. As suggested above, in conjunction with the manually actuateable steering means mounted to the handle, the sheath further includes at least two sets of steering wires which are flexible, but are coupled to the inner surface of the sheath near the distal end thereof. The steering wires may be axially translated relative to one another by actuation of the steering means, which action causes the sheath to bend and curve accordingly.

Referring now to one possible surgical instrument attachment the anastomosing and stapling attachment, this attachment comprises an anvil portion, and a staple, blade and reservoir portion, which includes a pair of turning drive shafts which are coupleable to the drive components of the shaft element described above, and a corresponding pair of advancing and retracting nuts mounted to the turning drive shafts, but which are prevented from rotating and therefore linearly advance and retract along the shafts when they tum.

The anvil portion is bullet shaped, having a blunt nosed top portion, a flat cutting support surface on the bottom, and a freely rotating coupling post extending axially from the bottom surface. This coupling post is designed to be selectively coupleable and removable from the corresponding nut mounted to one of the turning drive shafts.

The staple, blade, and reservoir portion (SBR portion) is cylindrical in shape, forming a housing which has a hollow interior. It is this hollow interior which forms the reservoir. On the axially outward facing surface of the cylindrical wall of the housing are a series of staple ports, through which the staples of the device are discharged. A series of staple drivers are mounted within the cylindrical walls, beneath the staple ports, for driving the staples therethrough. The blade is similarly cylindrical, and seats in the inside of the housing, against the inner surface of the wall thereof. Both the blade and the staple driver are mounted to the second nut, which is, in tum, mounted to the other turning drive shaft. As the tuning drive shaft rotates, the nut (which is constrained against rotating) advances along the shaft, thus linearly advancing the blade and staple driver. The blade and the staple driver are, therefore, selectively advanceable axially outward from the housing, in accordance with actuation of the appropriate trigger on the handle.

In a preferred embodiment set forth in the referenced application, the anvil portion and the SBR portion further comprise an electromagnetic sensor mechanism, coupled to the LCD indicator of the handle, which sensor is activated when the two portions have approached each other to the extent necessary for a safe staple firing, whereby the surgeon may have remote knowledge of the state of the attachment disposed within the colon.

An observed problem with prior art devices used in the anastomosing, stapling and resecting surgical procedure described above, relates to the best indications which the surgeon may remotely receive as to the conditions within the patients colon. For example, it is critical that the surgeon know whether the tissue being coupled forms a contiguous and sealed ring, such that the recoupled ends of the colon do not contain a hole through which bowel contents may leak into the body cavity. Post surgical infection rates due to such failures are a leading cause of complications and are often severe and are a leading cause of morbidity.

An associated issue of which surgeons who carry out these procedures must be cognizant relates to the ongoing viability of the tissue which has been resealed. A frequent post-surgical problem relates to the tissue necrosis which may occur if the staple ring is too tightly compressing the tissue, and preventing necessary blood flow thereto. A simple light-based mechanically measured distance indicator means is insufficient to avoid both of these problem Prior art devices fail to provide the means by which information regarding the state of the tissue being manipulated may be measured and used by the surgeon, and the instrument itself, to ensure a more positive outcome.

It shall be understood that this problem, i.e., the failure of remotely controlled surgical instruments to provide for the gathering, displaying, and influencing of automatic actions, of information critical to the success of the surgical procedure simultaneously with the action of the device, is not limited to the specific instances discussed above. Rather, this failure is prevalent throughout surgical instrumentation.

It is therefore a principle object of the present invention to provide a surgical system with an attachment which is remotely controlled and includes information-gathering sensors, communication and processing capacities, information storage capacity, and indicating means by which the user and/ or remote decision-making systems may choose to control the instrument and activate the features of the attachment in accordance with the gathered and relayed information.

Other objects of the present invention shall be recognized in accordance with the description thereof provided hereinbelow, and in the Detailed Description of Preferred Embodiments in conjunction with the remaining Figures.

The reader may be further enlightened as to the state of the art by reference to the publication identified as US 5 667 517 A. Essential features of the present invention in common with US 5 667 517 A are recited in the pre-characterizing part of claim 1. The present invention seems to alleviate at least some of the technical limitations of the prior art and accordingly provides a surgical system according to claim 1.

The preceding objects of the invention are provided by a surgical system as claimed. In a described arrangement there is an electromechanical driver, a flexible shaft, and remote surgical attachment including a controller processor unit mounted in the handle which is connected via cabling in the flexible shaft to a sensor and memory unit in the remote attachment. More particularly, with respect to the bowel surgery anastomosing, resecting, and stapling attachment described above in the Description of the Prior Art, the present invention shall be set forth with respect to the same application. Specifically, with respect to the sensor and memory unit mounted in the anastomosing, resecting, and stapling attachment, the sensor utilized is a pulse oximeter.

First, there is described a subsystem of a device which comprises three components, which are (1) an electromechanical driver, (2) a flexible shaft, and (3) an anastomosing, resecting, and stapling attachment.

First, with regard to the electromechanical driver, the handle has a pistol grip-styled design, having at least two finger-actuatable triggers which independently initiate motors which tum drive shafts mounted within the flexible shaft. The handle further includes a remote status indicator coupled to the processor unit in the handle. This indicator provides either visual, audio, or electrical output (to be output to a separate display device). The handle and flexible shaft further include a shaft steering means comprised of steering wires controlled by a handle mounted motor drive system including a manually actuatable steering means for directing the steering means, for example, a joystick or trackball.

In the electromechanical driver, the driver components are integrated with the controller components. It should be noted that other electromechanical drivers may comprise a driver unit which is physically separate from a controller unit. That is, the driver unit may comprise the above-described motors and the above-described steering means, and the controller unit may comprise the above-described triggers, the above-described remote status indicator, as well as the above-described manually actuatable steering means. The controller unit components communicate with the driver unit components by wireless transmission, for example, through infrared, radio waves, other electromagnetic waves, or ultrasound. In such a configuration, for example, the driver unit may be located out of the surgeon's arm's reach, while the controller unit may be selectively coupleable to that portion of the flexible shaft which is closer to the patient and closer to the surgeon. It should be further understood that the electromechanical driver assembly may comprise more than two separate units, and such units may each house only one, or more than one, of the above-described separate components, all communicating by wireless means as described above. For example, the remote status indicator described above could be part of a third unit which mounts to a visor wearable by the surgeon. It should be further understood that all communications between these components as described herein may take place by wireless means.

Second, with respect to the flexible shaft, the shaft comprises a tubular sheath, preferably formed of a simple elastomeric material which is tissue compatible and which is sterilizable (i.e., is sufficiently rugged to withstand an autoclave). Within the elastomeric sheath are a pair of smaller fixed tubes, each of which contain a flexible drive shaft which is capable of rotating within the tube. As suggested above, in conjunction with the steering means mounted in the handle, the sheath further includes at least two steering wires which are flexible, but are coupled to the inner surface of the sheath near the distal end thereof. In addition, the flexible shaft includes a least one electrical lead and corresponding coupling terminals at each end, for coupling to the processor and controller means in the handle with the sensor and memory components in the distally mounted surgical attachment.

Third, with regard to the anastomosing, resecting, and stapling attachment, a single example of the many alternative surgical attachments is now described. This attachment comprises a selectively advanceable and retractable anvil portion, and a staple, blade, sensor, and reservoir portion. This latter element includes the drive elements necessary to move the anvil forward and back, as well as the motive elements which drive the staples and blade through the tissue. These motive elements are coupleable to the drive components of the shaft element described above. Included in the staple, blade, sensor and reservoir portion, also, are a pulse oximeter sensor and a tissue proximity sensor, as well as a memory unit which contains important identification information which may be retrieved by the processor unit in the handle upon connection to the flexible shaft.

As stated above, when initially coupled to the flexible shaft (which shall be hereinafter taken to be permanently coupled to the handle unit), and the handle is powered up, the first internal act to take place is for the processor unit in the handle to query the memory unit in the attachment as to its identity and status. More particularly, as the anastomosing, resecting, and stapling attachments may come in different diameters, shapes, lengths, and stapling arrangement (as well as many other potential variations) which are readily readable by the handle mounted processor unit and displayed for the user either on a remote display panel to which the handle is coupled, or on a display screen which is integrally included in the handle itself.

The status of the attachment shall also be queried, for example, as to what the functionality of the attachment is, and whether or not it has been previously used, and therefore, no longer capable of firing a second time.

Once this information has been gathered, and it is determined that the attachment is the appropriate one, the surgical step for which this attachment is utilized may continue. In particular, the anvil tip is advanced via action of the drive motor and drive elements of the handle, shaft, and attachment, until it may be manually separated from the remainder of the attachment. The appropriate opening is made in the previously cut and stapled closed proximal end of the bowel and the anvil tip of the attachment is placed therein. The remainder of the attachment and the appropriate length of the flexible shaft is inserted through the rectum and up the bowel until the attachment coupling shaft is advanced through a small opening in the cut and stapled closed distal end of the bowel section.

After recoupling, the anvil portion is retracted toward the stapling portion until it is mechanically determined that the two portions are within the range which is appropriate for staple firing. At this time, the physician user is unable to simply remotely fire the staples. This lock out feature is maintained by the processor unit until such time as the surgeon causes the processor until in the handle to query the tissue proximity sensor and the pulse oximeter sensor in the attachment to determine if the tissues to be stapled together form a completely contiguous ring so that the two tubular sections of bowel can be properly mechanically united. This measurement may be made optically, or by any other suitable means, by which the transmission of some signal which should be blocked by the intervening tissue is received by an opposing sensor. The results of this query are then relayed back to the processor in the handle which carries out the appropriate analysis. If the proximity query results in a negative analysis outcome, an indicator light, audible alarm, or other suitable means of alerting the surgeon to this condition is provided. The processor will also disarm the stapling mechanism (by removing power to the drive mechanisms, for example) to prevent an attempted override by the surgeon.

If the proximity query results in a positive response, however, the next in the series of tests to determine if the surgeon may safely join the sections of bowel is carried out. Specifically, the processor in the handle activates the pulse oximeter sensor and queries it regarding whether the proper level of blood profusion remain in the tissue sections to be joined. As suggested above, if the stapling procedure has the effect of cutting off the necessary blood supply to sections of the joined tissue, a necrotic region will develop in the bowel, and future complications (which may be fatal) will arise. The results of this query are also relayed to the processor in the handle. Again, if the results of the processing of this information is negative, an indication of this state is provided by the processor. As above, if negative, the surgeon will be unable to mechanically fire the staples, as the processor will disarm the motor assembly.

Additional features and aspects of the present invention are set forth in greater detail in the description of the preferred embodiments provided hereinbelow.

### A Brief Description of the Drawings:

Figure 1 is a side perspective view of an anastomosing, resecting, and stapling instrument of the prior art;
Figure 2 is a side cross-section view of a handle and flexible shaft used in a system of a preferred embodiment of the present invention, wherein important internal features of the control systems are provided in detail;
Figure 3 is a side cut-away view of an anastomosing, resecting, sensing, and stapling attachment which is also used in a system embodying an aspect of the present invention; and
Figure 4 is a flow chart illustrating the logical sequence of processor, sensor, and mechanical actions which are illustrative of features of the system of a preferred embodiment of the present invention.

### A Detailed Description of the Preferred Embodiments:

While the present invention will be described more fully hereinafter with reference to the accompanying drawings, in which particular embodiments are shown, and with respect to methods of surgical use, it is to be understood at the outset that persons skilled in the art may modify the invention herein described while achieving the functions and results of this invention. Accordingly, the descriptions which follow are to be understood as illustrative and exemplary of specific structures, aspects and features within the broad scope of the present invention and not as limiting of such broad scope. Like numbers refer to similar features of like elements throughout.

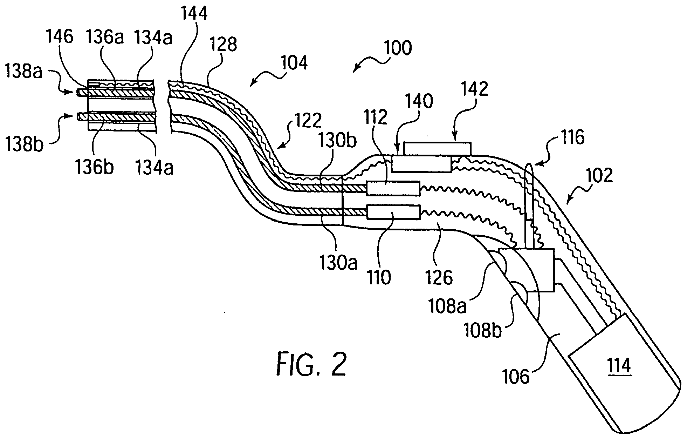

Referring now to Figure 2, with respect to the electromechanical driver 100, the driver has a handle portion 102 and a flexible drive shaft portion 104. The handle 102 includes a portion which is shaped in a manner which is easily gripped by the surgeon operator, for example, in the embodiment provided herein the handle comprises a pistol grip-styled portion 106. The grip portion 106 includes at least two, and in the present embodiment exactly two, independently finger actuateable triggers 108a, 108b. The finger triggers 108a, 108b are independently coupled to separate motors 110,112, housed within the interior volume of the handle 100. Each motor 110,112 turns a separate flexible drive shaft (described more fully, hereinbelow).

More particularly, with respect to the motors 110,112, each is a dual direction motor. In addition to being coupled to the finger-actuateable switch, the motors are also each separately coupled to a power source 114 (which is a common source in the
present embodiment) and a manual drive switch 116. The manual drive switch 116 is provided on the top of the handle 100, such that the surgeon operator can selectively alter the turning direction of each motor. In the preferred embodiment, the power source 114 supplying the motors 110,112 is a single direct current supplying removable and rechargeable battery pack. It shall be understood that alternative power sources, including dual direct current sources or single remote alternating current sources (such as the alternating current provided from standard United States 120 Volt, 60 Hertz wall outlets) may be used in conjunction with alternative embodiments. In the event that the driver device should be useable with an alternating current, either a transformer can be included between the motor and the power source, or a more sophisticated intermediate gearing assembly may be provided between the motor and the extended turning drive shaft.

In addition to the motors 110,112 components and the related power and drive switch elements, the handle 100 further incudes a motor driven shaft steering carriage assembly, which is coupled to a manual input means, which is coupled to steering steering wires in the flexible shaft for selectively steering the distal tip of the flexible drive shaft 122.

The handle also includes a processor element 140 and output display device 142 (mounted on the exterior of the handle). The processor element 140 and the display device 142 are each electrically coupled to the power source to provide electrical power to carry out their actions. The processor unit and display element are similarly coupled to one another to permit the processor to display the signal output generated thereby. The processor unit is also coupled via an electrical cable 144 to an input terminal 146 at the distal tip of the flexible shaft 122.

More particularly, with respect to the flexible shaft 122, the shaft comprises a tubular sheath 128 which is formed of a simple, tissue compatible, elastomeric material. As this device is to be reused, it is important that the material be sterilizable (i.e., is sufficiently rugged to withstand an autoclave). While the example illustrated comprises a contiguous handle 100 and shaft 122, it shall be understood that one having ordinary skill in the art may provide an alternative example having a separable handle and shaft, thus permitting alternative shaft lengths for alternative purposes. In such cases, the flexible shaft 122 and the handle 100 portions should in dude an interface between the proximal end of the shaft and the distal end of the handle which should include a coupling means for the drive components.

Specifically regarding the drive components 130a,130b of the shaft 122, within the elastomeric sheath 128 are a pair of smaller fixed tubes 134a,134b which each contain a flexible drive shaft 136a,136b which is capable of rotating within the corresponding tube 134a,134b. The flexible drive shaft 122, itself, simply must be capable of translating a torque from the motor in the handle to the distal end 138a,138b of the shaft 122, while still being flexible enough to be bent, angled, curved, etc. as the surgeon deems necessary to "snake" through the colon of the patient. For example, the drive shafts may comprise a woven steel fiber cable, a high tensile strength polymeric material, or a sufficiently flexible unitary metal shaft.

In order for the distal ends 138a, 138b of the drive shafts 136a, 136b to couple with an attachment, such as anastomosing, resecting, sensing, and stapling attachment, the distal tips 138a, 138b of the drive shafts must have a conformation which permits the continued translation of torque. In the present example, this coupling is achieved by a geometric fitting, and more precisely, the distal tips of the drive shafts are hexagonal, and thereby fit into a hexagonal recesses in the coupling interface of the attachment. In a preferred example, the attachment and the distal end of the shaft should include a collar, or other aligning means, for facilitating the fitting of the attachment onto the distal end of the shaft.

In addition, the shaft includes an electrical wire 144 extending from the end coupled to the handle to the end which couples to the surgical attachment. The first end of the shaft includes the terminal inputs which are coupled to the processor unit. The second end includes a terminal146 for coupling to the corresponding electrical input/ output of the surgical attachment (described more fully hereinbelow).

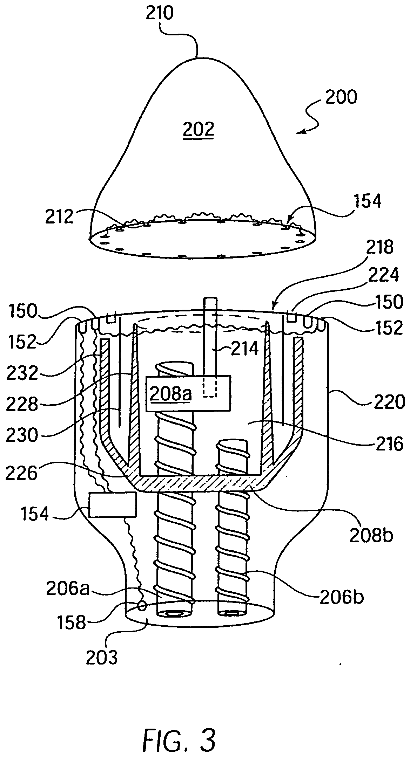

With reference now to Figure 3, a preferred example of the anastomosing and stapling attachment 200 is described. This attachment comprises an anvil portion 202, and a staple, blade, sensor, and reservoir (SBSR) portion 204, which includes a pair of turning drive shafts 206a,206b which are coupleable to the drive components 136a,136b of the driver component described above with reference to Figure 2, and a corresponding pair of advancing and retracting members 208a,208b mounted within tracks and to the turning drive shafts, which are thereby prevented from rotating and therefore linearly advance and retract along the shafts 206a,206b when they turn. The anvil portion includes a series of light detectors. The SBSR includes pulse oximeter and tissue proximity sensors 150,152 and a memory and signal transmitter and receiver member 154 which couples to the flexible shaft at the corresponding terminal thereon by way of an electrical coupler 158 shown.

The anvil portion 202 is bullet shaped, having a blunt nosed top portion 210, a flat cutting support surface 212 on the bottom, and a coupling post 214 extending axially from the bottom surface. This coupling post 214 mounts to the first advancing and retracting member 208a which is mounted within a linear track whereby rotation of the shaft 206a causes the member 208a and the anvil 202 coupled thereto to move axially, but not rotationally. The exterior surface of the face of the anvil which opposes the SBSR includes a series of light emitting diodes 154.

The staple, blade, sensor, and reservoir portion (SBSR) portion 204 is cylindrical in shape, forming a housing which has a hollow interior 216. It is this hollow interior which forms the reservoir. On the axially outward facing surface 218 of the cylindrical wall 220 of the housing are a series of staple ports, through which the staples 224 of the device are discharged. A unitary blade and cylindrical staple driver component 226 is seated within the housing. A circumferential pulse oximeter 150 is mounted in the housing on the outside rim of the housing, radially adjacent to the staple ports. A pulse oximeter is a simple device which shines a specific frequency of light through a section of tissue to measure the absorption rate of the light. Because oxygenated blood has a different color than does blood which is not fully oxygenated, the profusion of fresh blood through a tissue may be determined by this device. Therefore, the pulse oximeter comprises a series of light emitting elements 154 on the anvil and light sensors 150 mounted around the circumferential rim of the housing member. As shown, the components of the pulse oximeter are electrically connected via wires running through the coupling post of the anvil.

In addition, the attachment also includes a tissue proximity sensor 152, a portion of which is similarly mounted to the external rim of the housing, radially adjacent to the pulse oximeter 150 and the staple ports. In addition, the tissue proximity detector 152 comprises a series of simple light emitting elements 154 on the anvil (for example the same light emitting elements of the pulse oximeter), and a corresponding series of light detectors 152 mounted around the circumferential rim of the housing member. In the proximity sensor, if the tissue which is supposed to be disposed between the anvil and housing is present, then the proximity sensor's light detectors will not receive a signal (or at least below a set threshold}, alternatively, if the tissue is missing, then the light detectors will receive a stronger signal, indicating that there is no tissue blocking the transmission of the light.

More particularly, the blade and staple driver component comprises a single element having two concentric cylindrical portions. The blade portion 228 seats within the hollow interior 216, against the interior wall 230 thereof. The staple driver portion 232 seats within the wall 230 of the SBSR portion and includes a series of outwardly projecting protuberances which push against staples mounted within the staple ports.

The blade 228 and staple driver portions 232 are coupled at the interior end thereof to a threaded member 208b which seats around turning shaft 206b. The threaded member 208b is constrained within a linear track so that the blade and staple driver are advanced linearly upon rotation of the turning shaft 206b.

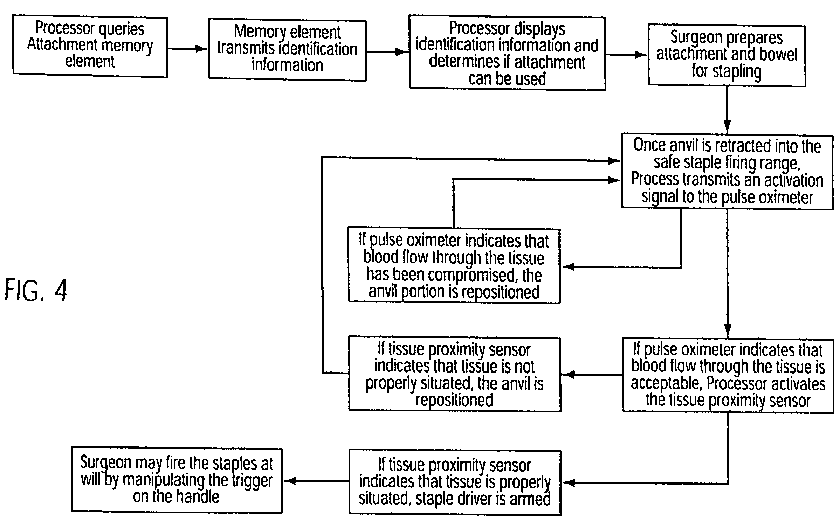

In practice and with reference to the flow chart provided in Figure 4, this attachment and the associated controller features of the system of the present invention are utilized once the section of the colon to be removed has been resected and the two opposing ends of the adjacent bowel have been stapled shut. The surgeon begins by coupling the anastomosing, resecting, sensing, and stapling attachment 200 to the distal end of the flexible shaft. The processor in the handle unit queries the memory element in the attachment and requests identification information. The attachment transmits the requested information, including size, status and functionality information. The processor in the handle outputs this information to the display panel, for visual inspection by the surgeon user. The processor further establishes whether the surgeon may activate the driver means in correspondence to whether the information received from the attachment was acceptable (i.e., is it unused and functional).

In the event that the identification information received is acceptable, the surgeon then triggers the electromechanical driver to advance the anvil portion 202 to its fullest extent. The anvil head 202 is then decoupled from the first advancing and retracting member 208 and inserted into the stapled proximal end of the bowel (which is then opened partially to receive the anvil head). The proximal end of the bowel is then sutured closed. The surgeon then advances the shaft 206a and the SBSR portion 204 of the attachment up the colon until it extends through the stapled distal end of the colon. (Alternatively, the surgeon may advance only the flexible shaft up the colon and then reattach the SBSR portion to the distal end once it emerges from the distal end of the bowel) The surgeon then couples the anvil 202 to the advancing and retracting member 208a by rotation of the corresponding drive shaft 136a. Subsequent reverse biasing and triggering of the same motor in the handle 100 causes the anvil 202 to retract toward the SBRS portion 204.

Once retracted into the safe staple firing range, the processor disables the staple firing motor and again queries the attachment. In fact, the processor in the handle transmits an activation signal to the pulse oximeter, to sense the blood profusion through the tissue compressed between the anvil and SBSR portions. If the response received from the sensor is that the blood flow through the tissue has been compromised, the anvil portion is repositioned and the process begins anew. If the response received from the sensor is that the blood flow is acceptable, then the processor activates the tissue proximity sensor. If the response is that the tissue is properly situated, then the staple driver is armed and the surgeon can fire the staples at will by manipulating the trigger on the handle. If the proximity detector response is negative, the anvil is repositioned.

While there has been described and illustrated new and novel electromagnetic driver mechanisms having an attachment processor controller, for use with surgical attachments in a surgical system as claimed.

## Claims

1. A surgical system comprising:
a torque generating element (110, 112);
a torque translating member (208a, 208b) that is coupleable at a first end to the torque generating element; and
a surgical tool head (200, 210, 220) including at least one selectively moveable element (214) that is coupled to a second end of the torque translating member and that is selectively moveable by the torque generating member via the torque translating member,
the surgical tool head further including a sensor mechanism (150, 152) configured to sense and provide data corresponding to at least one feature of an environment surrounding the surgical tool head, and
a processor element (154) able to electrically communicate to receive said sensor data,
**characterized in that** said surgical tool head (200,210,220) is provided by a surgical attachment adapted to be coupled to the torque translating member (208a,208b) by a surgeon, and **in that** said processor element (154) is responsive to said sensor data to control the application of an amount of torque to be generated by the torque generating element to correspond to the data.

2. The surgical system as set forth in claim 1, wherein the surgical attachment includes a stapling instrument.

3. The surgical system as set forth in claim 2, wherein the stapling instrument comprises: a first jaw; a second jaw coupled to the first jaw, wherein the at least one selectively moveable element includes at least one of the first and second jaws.

4. The surgical system as set forth in claim 3, wherein the first and second jaws are moveable relative to each other so as to staple a section of tissue disposed between the first and second jaws.

5. The surgical system as set forth in claim 4, wherein the sensor mechanism is configured to sense and provide data corresponding to a condition of the section of tissue when the section of tissue is disposed between the first and second jaws.

6. The surgical system as set forth in any of claims 1-5, wherein the sensor mechanism includes a pulse oximeter (150).

7. The surgical system as set forth in any of claims 1-6, wherein the sensor mechanism includes a tissue proximity detector (150).

8. The surgical system as set forth in any preceding claim, further comprising a display device (142) configured to provide a display that corresponds to the data.

9. The surgical system as set forth in any preceding claim, wherein the sensor mechanism is selectively activateable.

10. The surgical system as set forth in any of claims 1-8, wherein the torque translating member includes a rotatable drive shaft (136) housed within a flexible shaft (134).

11. The surgical system as set forth in claim 10, wherein the flexible shaft (134) is coupleable at a first end to an electromechanical driver element {110, 112), the torque generating element housed within the driver element (110, 112).

12. The surgical system as set forth in claim 10, wherein, when the flexible shaft (134) is coupled at its first end to the driver element (I 10, 112), the first end of the torque translating member is caused to be coupled to the torque generating element.

13. The surgical system as set forth in claim 12, wherein the flexible shaft (134) is coupleable at a second end to a terminal of the surgical attachment.

14. The surgical system as set forth in claim 13, wherein, when the flexible shaft (134) is coupled at its second end to the terminal of the surgical attachment, the second end of the torque translating member is caused to be coupled to the at least one selectively moveable element.

15. The surgical system as set forth in any preceding claim wherein the surgical system is an anastomosing, resecting and stapling instrument.

## Patentansprüche

1. Chirurgisches System, umfassend:
ein drehmomenterzeugendes Bauteil (110, 112);
ein drehmomentübertragendes Bauteil (208a, 208b) das an einem ersten Ende mit dem drehmomenterzeugenden Bauteil koppelbar ist; und
einen chirurgischen Werkzeugkopf (200, 210, 220), der mindestens ein selektiv bewegbares Bauteil (214) aufweist, das an ein zweites Ende des drehmomentübertragenden Bauteils gekoppelt ist und das selektiv durch das drehmomenterzeugende Bauteil über das drehmomentübertragende Bauteil bewegbar ist, wobei der chirurgische Werkzeugkopf ferner einen Sensormechanismus (150, 152) aufweist, der konfiguriert ist, Daten bezüglich mindestens eines Merkmals einer Umgebung, die den Werkzeugkopf umgibt, zu erfassen und zu übertragen, und
ein Prozessorbauteil (154), das in der Lage ist, zum Empfangen von Sensordaten elektrisch zu kommunizieren,
**dadurch gekennzeichnet, dass**
der chirurgische Werkzeugkopf (200, 210, 220) mit einem chirurgischen Vorsatzteil versehen ist, das angepasst ist, an das drehmomentübertragende Bauteil (208a, 208b) durch einen Chirurgen gekoppelt zu werden, und dadurch, dass
das Prozessorbauteil (154) auf die Sensordaten zum Steuern der Anwendung einer mit den Daten übereinstimmenden Drehmomentgröße, die durch das drehmomenterzeugende Bauteil zu erzeugen ist, reagiert.

2. Chirurgisches System wie in Anspruch 1 angegeben, wobei das chirurgische Vorsatzteil ein heftendes Instrument umfasst.

3. Chirurgisches System wie in Anspruch 2 angegeben, wobei das heftende Instrument umfasst: einen ersten Klemmbacken, einen zweiten Klemmbacken, der mit dem ersten Klemmbacken gekoppelt ist, wobei das mindestens eine selektiv bewegliche Bauteil mindestens einen der ersten und zweiten Klemmbacken umfasst.

4. Chirurgisches System wie in Anspruch 3 angegeben, wobei die ersten und zweiten Klemmbacken relativ zueinander beweglich sind, um so einen Gewebeabschnitt zu heften, der zwischen dem ersten und dem zweiten Klemmbacken angeordnet ist.

5. Chirurgisches System wie in Anspruch 4 angegeben, wobei der Sensormechanismus konfiguriert ist, Daten zu erfassen und bereitzustellen, die einem Zustand des Gewebeabschnitts entsprechen, wenn der Gewebeabschnitt zwischen den ersten und zweiten Klemmbacken ist.

6. Chirurgisches System wie in einem der Ansprüche 1 bis 5 angegeben, wobei der Sensormechanismus ein Pulsoximeter (150) umfasst.

7. Chirurgisches System wie in einem der Ansprüche 1 bis 6 angegeben, wobei der Sensormechanismus einen Gewebenäherungsdetektor (150) umfasst.

8. Chirurgisches System wie in einem der vorhergehenden Ansprüche angegeben, ferner umfassend eine Anzeigevorrichtung (142), die konfiguriert ist, eine den Daten entsprechende Anzeige bereitzustellen.

9. Chirurgisches System wie in einem der vorhergehenden Ansprüche angegeben, wobei der Sensormechanismus selektiv aktivierbar ist.

10. Chirurgisches System wie in einem der Ansprüche 1 bis 8 angegeben, wobei das drehmomentübertragende Bauteil eine innerhalb einer flexiblen Welle (134) untergebrachte drehbare Antriebswelle (136) aufweist.

11. Chirurgisches System wie in Anspruch 10 angegeben, wobei die flexible Welle (134) an einem ersten Ende an ein elektromechanisches Antriebsbauteil (110, 112) koppelbar ist, wobei das drehmomentübertragende Bauteil innerhalb des Antriebsbauteils (110, 112) untergebracht ist.

12. Chirurgisches System wie in Anspruch 10 angegeben, wobei, wenn die flexible Welle (134) an ihrem ersten Ende an das Antriebsbauteil (110, 112) gekoppelt ist, bewirkt wird, dass das erste Ende des drehmomentübertragenden Bauteils mit dem drehmomenterzeugenden Bauteil gekoppelt wird.

13. Chirurgisches System wie in Anspruch 12 angegeben, wobei die flexible Welle (134) an einem zweiten Ende an ein Anschlusselement des chirurgischen Vorsatzteils koppelbar ist.

14. Chirurgisches System wie in Anspruch 13 angegeben, wobei, wenn die flexible Welle (134) an ihrem zweiten Ende an das Anschlusselement des chirurgischen Vorsatzteils gekoppelt ist, bewirkt wird, dass das zweite Ende des drehmomentübertragenden Bauteils mit dem mindestens einen selektiv beweglichen Bauteil gekoppelt wird.

15. Chirurgisches System wie in einem der vorhergehenden Ansprüche angegeben, wobei das chirurgische System ein anastomosierendes, resektierendes und heftendes Instrument ist.

## Revendications

1. Système chirurgical comprenant :
un élément générateur de couple (110, 112) ;
un élément de transfert de couple (208a, 208b) qui peut être couplé à une première extrémité à l'élément générateur de couple ;
une tête d'outil chirurgical (200, 210, 220) comprenant au moins un élément sélectivement mobile (214) qui est couplé à une seconde extrémité de l'élément de transfert de couple et qui est sélectivement déplaçable par l'élément générateur de couple via l'élément de transfert de couple,
la tête d'outil chirurgical comprenant en outre un mécanisme de capteur (150, 152) configuré pour capter et fournir des données correspondant à au moins une caractéristique d'un environnement entourant la tête d'outil chirurgical, et un élément de processeur (154) qui est à même de communiquer électriquement pour recevoir lesdites données du capteur,
**caractérisé en ce que** :
ladite tête d'outil chirurgical (200,210,220) est fournie par une attache chirurgicale adaptée pour être couplée à l'élément de transfert de couple (208a, 208b) par un chirurgien et **en ce que**
ledit élément de possesseur (154) est sensible auxdites données du capteur pour commander l'application d'une quantité de couple à générer par l'élément générateur de couple pour correspondre aux données.

2. Système chirurgical selon la revendication 1, dans lequel l'attache chirurgicale comprend un instrument d'agrafage.

3. Système chirurgical selon la revendication 2, dans lequel l'instrument d'agrafage comprend un premier mors ; un second mors couplé au premier mors, dans lequel le au moins un élément sélectivement mobile comprend au moins l'un des premier et second mors.

4. Système chirurgical selon la revendication 3, dans lequel les premier et second mors peuvent être déplacés l'un par rapport à l'autre de manière à agrafer une section de tissu disposée entre les premier et second mors.

5. Système chirurgical selon la revendication 4, dans lequel le mécanisme de capteur est configuré pour capter et fournir des données correspondant à un état de la section de tissu lorsque la section de tissu est disposée entre le premier et le second mors.

6. Système chirurgical selon l'une quelconque des revendications 1 à 5, dans lequel le mécanisme de capteur comprend un oxymètre d'impulsion (150).

7. Système chirurgical selon l'une quelconque des revendications 1 à 6, dans lequel le mécanisme de capteur comprend un détecteur de proximité de tissu (150).

8. Système chirurgical selon l'une quelconque des revendications précédentes, comprenant en outre un dispositif d'affichage (142) configuré pour fournir un affichage qui correspond aux données.

9. Système chirurgical selon l'une quelconque des revendications précédentes, dans lequel le mécanisme de capteur peut être activé sélectivement.

10. Système chirurgical selon l'une quelconque des revendications 1 à 8, dans lequel l'élément de transfert de couple comprend un arbre d'entraînement rotatif (136) logé dans un arbre flexible (134).

11. Système chirurgical selon la revendication 10, dans lequel l'arbre flexible (134) peut être couplé à une première extrémité à un élément d'entraînement électromécanique (110, 112), l'élément générateur de couple étant logé dans l'élément d'entraînement (110,112).

12. Système chirurgical selon la revendication 10, dans lequel, lorsque l'arbre flexible (134) est couplé à sa première extrémité à l'élément d'entraînement (110, 112), la première extrémité de l'élément de transfert de couple est amenée à être couplée à l'élément générateur de couple.

13. Système chirurgical selon la revendication 12, dans lequel l'arbre flexible (134) peut être couplé à une seconde extrémité à une borne de l'attache chirurgicale.

14. Système chirurgical selon la revendication 13, dans lequel, lorsque l'arbre flexible (134) est couplé à sa seconde extrémité à la borne de l'attache chirurgicale, la seconde extrémité de l'élément de transfert de couple est amenée à être couplée au au moins un élément sélectivement mobile.

15. Système chirurgical selon l'une quelconque des revendications précédentes, dans lequel le système chirurgical est un instrument d'anastomose, de résection et d'agrafage.
